# EUROPEAN PATENT APPLICATION

(11) **EP 4 790 151 A1**
(43) Date of publication of application: **12.08.2026**
(21) Application number: 25382105.2
(22) Date of filing: 07.02.2025
(51) Int. Cl.: C12Q 1/6886

(54) **DIAGNOSIS AND/OR PROGNOSIS OF POLYCYSTIC OVARY SYNDROME**

(71) Applicant: Fundación para la Investigación Biomédica del Hospital Universitario Ramón y Cajal, 28034 Madrid (ES); Universidad De Alcalá De Henares, 28801 Alcalá De Henares (ES); Consorcio Centro de Investigación Biomédica en Red, 28029 Madrid (ES)
(72) Inventor: ESCOBAR MORREALE, Héctor Francisco, 28034 Madrid (ES); LUQUE RAMÍREZ, Manuel, 28034 Madrid (ES); INSENSER NIETO, María Rosa, 28034 Madrid (ES); MARTÍNEZ GARCÍA, María Ángeles, 28034 Madrid (ES)
(74) Representative: Hoffmann Eitle

(57) **Abstract**

The present invention refers to the medical field. Particularly, the present invention refers to an *in vitro* method for the diagnosis and/or prognosis of polycystic ovary syndrome (PCOS).

## Description

### FIELD OF THE INVENTION

The present invention refers to the medical field. Particularly, the present invention refers to an *in vitro* method for the diagnosis and/or prognosis of polycystic ovary syndrome (PCOS).

### STATE OF THE ART

Despite a recent international consensus on assessment and management of the PCOS, the process of diagnosing a woman with PCOS in clinical practice still presents with unsolved challenges. For instance, the cut-off values for defining hirsutism using clinical scores are controversial. Furthermore, the widespread implementation of reliable assays for androgen measurement - i.e.: mass spectrometry (MS)-based methods - is still pending in most routine laboratories worldwide; the use of less accurate immunoassays carries a certain risk of misdiagnoses. In the same vein, the clinical definition of ovulatory dysfunction varies between scientific societies, and establishing the presence of oligo-ovulation accurately may be time-consuming in women with anovulatory but regular menstrual cycles. Not to mention that the most accurate diagnostic marker for polycystic ovarian morphology (PCOM) - namely follicle number per ovary - suffers from poor reproducibility between ultrasound operators even if counting is performed in real time and may lead to an overdiagnosis of PCOM.

Considering this background, there is an unmet medical need of biomarkers that properly identify women with PCOS and may contribute to solve present diagnostic limitations in the clinical setting.

The present invention is focused on solving this problem and an innovative method for the diagnosis and/or prognosis of PCOS is herein provided.

### DESCRIPTION OF THE INVENTION

### Brief description of the invention

The present invention refers to an *in vitro* method for the diagnosis and/or prognosis of PCOS, since the process of diagnosing a woman with PCOS in clinical practice is still being challenged by the lack of standardization of the signs, methods, and threshold values used to establish the presence of its individual diagnostic criteria.

Women with PCOS may present with a differential pattern of several novel circulating biomarkers such as miRNAs and low-molecular weight metabolites.

Consequently, the inventors of the present invention have designed a diagnostic study involving serum samples and clinical data from 253 premenopausal women diagnosed with PCOS and 71 non-hyperandrogenic control women, phenotyped by state-of-the-art methodology.

Women with PCOS were diagnosed using liquid chromatography-tandem mass spectrometry (LC-MS/MS) for quantifying biochemical hyperandrogenism and anti-Müllerian hormone (AMH) immunoassay or sonography for assessment of PCOM. The control group was comprised of premenopausal female volunteers of similar body mass index, with no signs of hyperandrogenism, reporting regular menses, and were assessed using the same methods used to diagnose PCOS in patients. The inventors used miRCURY LNA miRNA PCR assays to analyse two circulating miRNAs (miR-142-3p and miR-598-3p) that had shown a strong association with PCOS in a validation study previously conducted by their group. Serum samples were also submitted to proton nuclear magnetic resonance spectroscopy (¹H-NMRS) metabolomics profiling. The inventors studied diagnostic accuracy using binary logistic regression models and receiver operating characteristics (ROC) curve analyses.

The inventors of the present invention have identified that circulating miR-142-3p was underexpressed in plasma samples of women with PCOS compared to non-hyperandrogenic control women. On the contrary, miR-598-3p expression was similar between women with PCOS and non-hyperandrogenic controls. After adjusting by age, women with PCOS presented with higher fasting circulating concentrations of alanine, leucine, isoleucine, glutamic acid, threonine, acetate and lactate, and lower concentrations of glutamine and acetone, compared to non-hyperandrogenic control women. Abinomial logistic regression [χ² (10) = 94.2, P < 0.001] correctly classified women with PCOS or non-hyperandrogenic control women in 81% of cases. Sensitivity was 85.9%, specificity 60.0%, positive predictive value was 91.0%, and negative predictive value was 47.4%. Of the 10 predictive variables tested by the model, only four reached statistical significance: miR-142-3p, isoleucine, acetate, and AMH. The overall discriminatory ability of these variables was further addressed by ROC analyses. The model's area under the ROC curve had a confidence interval of 0.826 to 0.917. When the inventors analyzed the performance of the model analyzing separately non-obese and obese women, it correctly classified 82% and 90% of cases, respectively. When the analysis was restricted to women with hyperandrogenic phenotypes, non-ovulatory phenotypes, and non-hyperandrogenic phenotypes, the model correctly classified 82%, 84%, and 73% of cases.

In summary, women with PCOS in this series consistently underexpressed miR-142-3p in plasma and presented with a differential pattern of serum low-molecular weight metabolites, compared to non-hyperandrogenic women. These novel circulating biomarkers combined with follicular-phase anti-Müllerian hormone (AMH) levels, showed an excellent performance in identifying women with all PCOS phenotypes.

So, the first embodiment of the present invention refers to an *in vitro* method for the diagnosis and/or prognosis of polycystic ovary syndrome, which comprises assessing the expression level of the microRNA-142-3p in combination with the assessment of the amount of at least a metabolite selected from: isoleucine and/or acetate, in a biological sample obtained from the subject; alternatively, the present invention refers to the *in vitro* use of the microRNA-142-3p in combination with isoleucine and/or acetate, or of a kit comprising reagents for assessing the expression level of the microRNA-142-3p in combination with the assessment of the amount of a metabolite selected from: isoleucine and/or acetate, for the diagnosis and/or prognosis of polycystic ovary syndrome.

In a preferred embodiment, the present invention comprises assessing the expression level of the microRNA-142-3p in combination with the assessment of the amount of the metabolites isoleucine and acetate.

In a preferred embodiment, the present invention further comprises assessing the concentration of anti-Müllerian hormone.

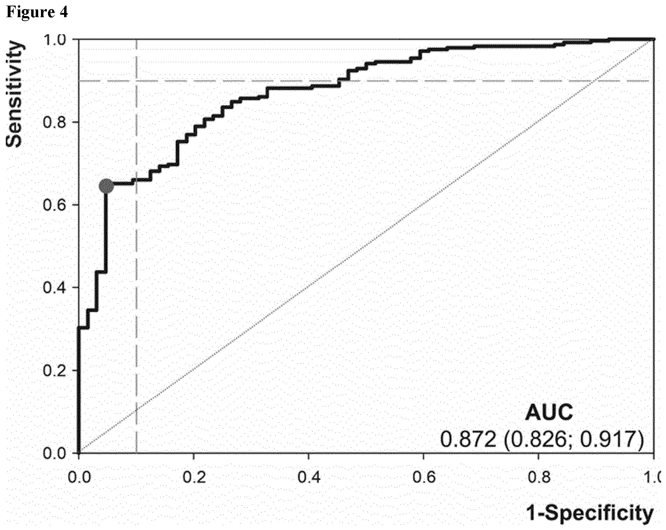

In a preferred embodiment, such as it is indicated in **Figure 4****,** the present invention comprises assessing the expression level of the microRNA-142-3p in combination with the assessment of the amount of the metabolites isoleucine and acetate in combination with the assessment of the concentration of anti-Müllerian hormone.

In a preferred embodiment, the identification of a lower level of expression of microRNA-142-3p in combination with a higher amount of isoleucine and/or acetate, as compared with a pre-established threshold value measured in control subjects who are not suffering from polycystic ovary syndrome, is an indication that the subject is suffering from polycystic ovary syndrome or has a poor prognosis.

In a preferred embodiment, the biological sample is selected from: blood, serum or plasma.

The second embodiment refers to a kit, suitable for the diagnosis and/or prognosis of polycystic ovary syndrome, which comprises reagents for assessing the expression level of the microRNA-142-3p in combination with reagents for the assessment of the amount of at least a metabolite selected from: isoleucine and/or acetate.

In a preferred embodiment, the kit further comprises reagents for assessing the concentration of anti-Müllerian hormone.

In a preferred embodiment, the kit comprises reagents for assessing the expression level of the microRNA-142-3p in combination with reagents for the assessment of the amount of the metabolites isoleucine and acetate, and reagents for the assessment of the concentration of anti-Müllerian hormone.

The third embodiment of the present invention refers to a hormonal treatment, insulin-sensitizing agents, anti-androgens, ovulation induction agents and/or statins for use in a method for treating polycystic ovary syndrome, wherein the method comprises a step before the treatment which comprises diagnosing polycystic ovary syndrome by following the method of the invention.

In a preferred embodiment, the hormonal treatment comprises estrogen and progestin or progestin-only medications; the insulin-sensitizing agents that comprise metformin; the anti-androgens that comprise spironolactone, finasteride or flutamide; and the ovulation induction agents that comprise clomiphene citrate, letrozole or gonadotropins.

Finally, the present invention also contemplates the possibility of stratifying patients suffering from polycystic ovary syndrome by utilizing the biomarkers or signatures of the invention, thereby enabling the personalization of treatment. Such stratification may be based on biomarker expression levels or amounts, specific biomarker profiles, or their correlation with clinical parameters, thereby enabling the personalization of treatment according to the patient's molecular and clinical characteristics. In other words, such stratification may categorize patients as responders or non-responders to existing treatments. This approach enables the personalization of treatment by selecting the most appropriate therapeutic strategy for each patient. As indicated above, the treatment may comprise hormonal treatment, insulin-sensitizing agents, anti-androgens, ovulation induction agents, and/or statins (non-exhaustive list), or any other treatment that could be beneficial for a patient suffering from polycystic ovary syndrome, such as the treatments disclosed in the following reviews [Luque-Ramirez M, Ortiz-Flores AE, Nattero-Chavez L, Escobar-Morreale HF. A safety evaluation of current medications for adult women with the polycystic ovarian syndrome not pursuing pregnancy. Expert Opin Drug Saf. 2020;19(12):1559-1576. doi:10.1080/14740338.2020.1839409*]* and *[*Luque-Ramirez M, Escobar-Morreale HF. Targets to treat androgen excess in polycystic ovary syndrome. Expert Opin Ther Targets. 2015;19(11):1545-1560. doi: 1 0.1517/14728222.2015.1075511*].*

For the purpose of the present invention the following terms are defined:
- According to the present invention, a reference value can be a "pre-established threshold value" or a "cut-off' value. Typically, a "threshold value" or "cut-off value" can be determined experimentally, empirically, or theoretically. According to the present invention, the "pre-established threshold" value refers to a value previously determined in subjects who are non-suffering from PCOS. A "threshold value" can also be arbitrarily selected based upon the existing experimental and/or clinical conditions, as would be recognized by a person ordinary skilled in the art. The "threshold value" has to be determined in order to obtain the optimal sensitivity and specificity according to the function of the test and the benefit/risk balance (clinical consequences of false positive and false negative). Typically, the optimal sensitivity and specificity (and so the "threshold value") can be determined using a Receiver Operating Characteristic (ROC) curve based on experimental data.
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of'. Thus, the phrase "consisting of" indicates that the listed elements are required or mandatory, and that no other elements may be present.

### Description of the figures

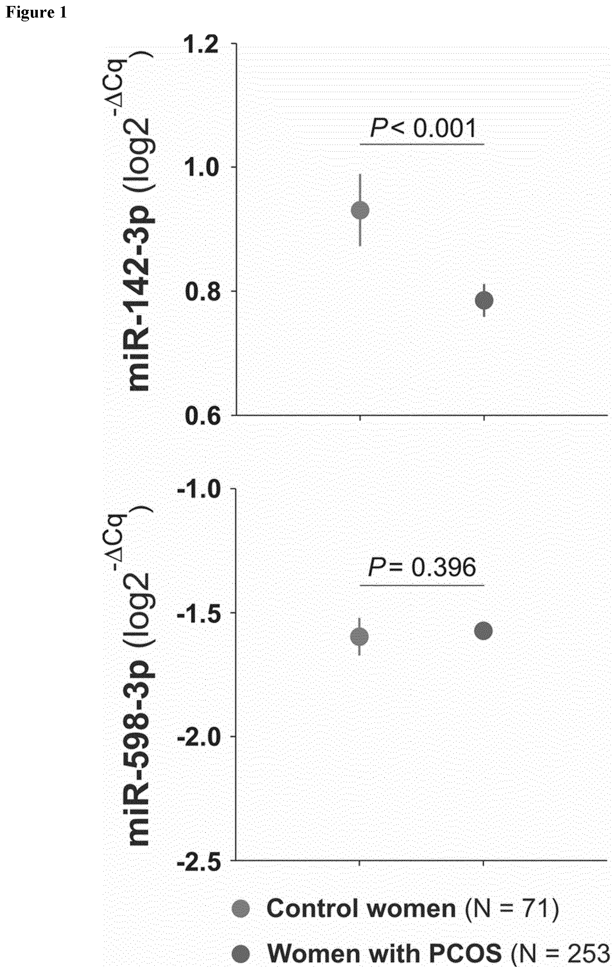

**Figure 1****.** Relative expression of each miRNA in control women and women with polycystic ovary syndrome. Data are expressed as means and 95% CI for the mean of log2^{-ΔCq}. For comparisons, data were submitted to a general linear model introducing age as a covariate. *P < 0.05.

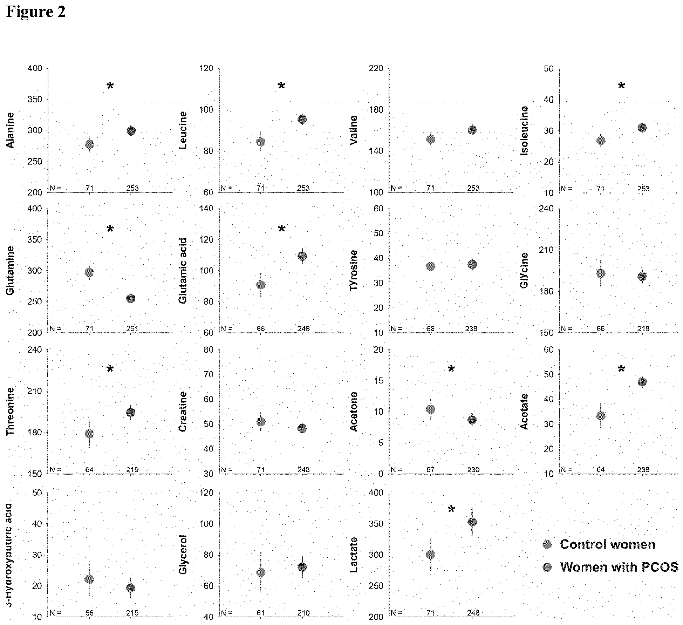

**Figure 2****.** Proton nuclear magnetic resonance spectroscopy metabolomics profiling in control women and women with PCOS. Data are shown as means (95% CI) of their µM concentrations. A general linear model was used for comparisons, introducing age as a covariate. The figures above the X-axis indicate available results, or in other words, those metabolites that did not fall below the detection threshold and were quantifiable with sufficient accuracy.

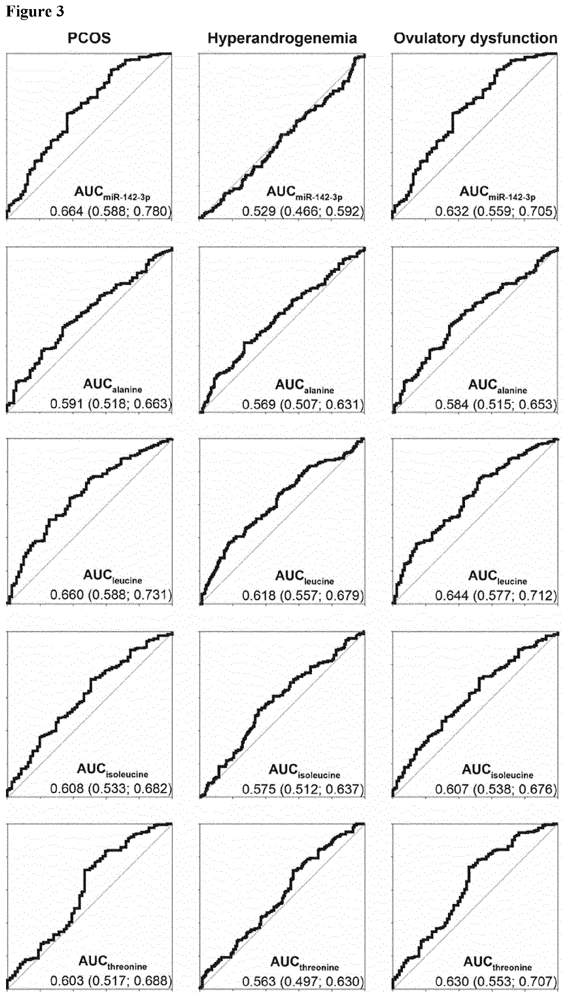

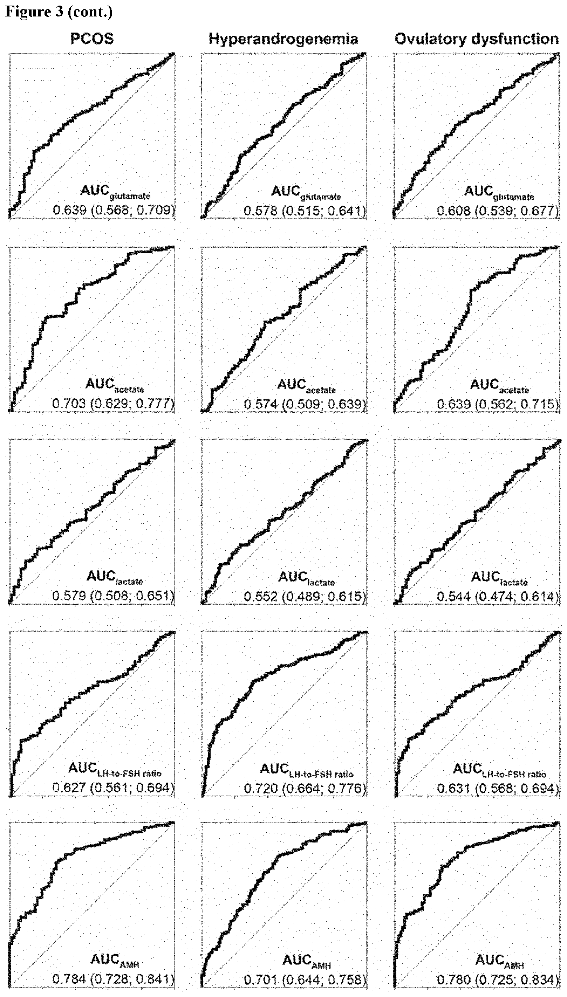

**Figure 3****.** Receiver-operating characteristics (ROC) curve analyses for microRNA-142-3p, low-molecular weight metabolites, and biochemical variables with statistically significant differences between women with PCOS and controls. The area under the ROC curve [AUC_{ROC} (95% CI)] indicates the probability of each biomarker of correctly identifying PCOS, hyperandrogenemia, or ovulatory dysfunction, respectively. The diagonal reference line in red corresponds to an AUC_{ROC} of 0.5 that suggests no discrimination.
**Figure 4****.** Receiver operating characteristic (ROC) curve analysis displaying the combined diagnostic accuracy of miR-142-3p, isoleucine, acetate, and anti-Müllerian hormone. The area under the ROC curve [AUC_{ROC} (95% CI)] indicates the ability of the model for discriminating between control women and women with PCOS. The diagonal reference line in red corresponds to an AUC_{ROC} of 0.5 that suggests no discrimination. The red point represents the best diagnostic accuracy. The horizontal green dotted line corresponds to 90% sensitivity. The vertical blue dotted line corresponds to 90% specificity.

### Detailed description of the invention

The present invention is illustrated by means of the Examples set below without the intention of limiting its scope of protection.

### Example 1. Material and methods

### Example 1.1. Subjects

The inventors used serum samples and clinical data from 340 consecutive premenopausal women referred to the Reproductive Endocrinology clinic because of symptoms of functional androgen excess or hyperandrogenemia. These individuals had been diagnosed with PCOS by state-of-the-art methodology including LC-MS/MS assays for diagnosing biochemical hyperandrogenism and anti-Müllerian hormone (AMH) immunoassays or sonography for assessment of PCOM. For LC-MS/MS measurements and AMH assay, biochemical hyperandrogenism and upper limit of normality, respectively, were established by the presence of values > 95th percentile from a sample of control women below detailed. World Health Organization group II ovulatory dysfunction (OD) (https://www.ncbi.nlm.nih.gov/books/NBK327781/) was defined by the presence of more than six cycles longer than 36 days in the previous year, absence of menstruation for three consecutive months, or by luteal phase progesterone concentrations below 17 µM in women with regular menses. As controls, the inventors included a group of 91 premenopausal female volunteers composed of hospital's staff, and overweight or obese women seeking advice solely for weight loss at the Department. Controls presented with no signs of hyperandrogenism and reported regular menses and were assessed using the same methods used to diagnose PCOS in patients. Those control women were similar in terms of body mass index (BMI) and age to the study population of hyperandrogenic women. Patients or controls had no history of oophorectomy or hysterectomy, nor had received treatment with hormonal contraceptives, anti androgens, or insulin sensitizers for at least 6 months before sampling.

Then, the inventors tested sera for haemolysis by measuring the miR-23a-3p to miR-451a ratio in all samples. Plasma samples suggesting haemolysis because of a ΔCq of more than 7.5 were excluded from subsequent analyses. Accordingly, 253 women with PCOS and 71 non-hyperandrogenic controls were finally included in the present study (**Table 1**).

**Table 1. Anthropometric, sex steroids and insulin sensitivity profile of women with PCOS and non-hyperandrogenic control women.**

| | | **Control women** | | | **Women with PCOS** | | | ***P*** |
|---|---|---|---|---|---|---|---|---|
| | | (n = 71) | | | (n = 253) | | | |
| *Age, years* | | 29 | ± | 6 | 27 | ± | 6 | 0.031 |
| *Body mass index, kg*/*m²* | | 26.6 | ± | 7.5 | 27.2 | ± | 7.6 | 0.199 |
| *Obesity, n (%)* | | 22 (31) | | | 84 (33) | | | 0.725 |
| *Waist circumference, cm* | | 82 | ± | 16 | 83 | ± | 18 | 0.468 |
| *Waist-to-hip ratio* | | 0.80 | ± | 0.12 | 0.81 | ± | 0.09 | 0.154 |
| *Insulin sensitivity index* | | 7.0 | ± | 3.5 | 6.3 | ± | 4.7 | 0.342 |
| *Anti-Müllerian hormone, pM^{f}* | | 22.7 | ± | 15.7 | 50.4 | ± | 37.5 | <0.001 |
| *LH-to-FSH ratio* | | 1.2 | ± | 1.4 | 1.5 | ± | 1.0 | 0.085 |
| *Total testosterone, nM^{f}* | | 0.9 | ± | 0.3 | 1.2 | ± | 0.6 | <0.001 |
| *Sex hormone-binding globulin, nM* | | 55 | ± | 26 | 45 | ± | 27 | 0.014 |
| *Calculated free testosterone, pM* | | 14.1 | ± | 5.4 | 24.1 | ± | 12.9 | <0.001 |
| *Androstenedione, nM* | | 4.6 | ± | 1.4 | 6.7 | ± | 2.7 | <0.001 |
| *DHEA-S, µM* | | 4.8 | ± | 1.9 | 6.6 | ± | 3.3 | <0.001 |
| *Estradiol, pM* | | 220 | ± | 198 | 151 | ± | 114 | 0.002 |
| *Estrone, pM* | | 166 | ± | 93 | 185 | ± | 107 | 0.093 |
| *PCOS phenotype* | | | | | | | | |
| | *Classic* | - | | | 184 (73) | | | - |
| | *Ovulatory* | - | | | 13 (5) | | | - |
| | | *Normoandrogenic* | - | | 56 (22) | | - | |

| | | | | | | | | |
|---|---|---|---|---|---|---|---|---|
| *Abbreviations, DHEA-S; dehydroepiandrosterone-sulphate; FSH, follicle-stimulating hormone; LH, luteinizing hormone; PCOS, polycystic ovary syndrome.* *Data are means* ± *SD or counts (percentages). A general linear model was used for comparisons adjusting by age. Free testosterone was calculated by Vermeulen 's formula using a default albumin level of 43 g*/*L (Vermeulen et al*., *1999).* | | | | | | | | |

All patients and controls provided informed consents allowing the inventors to include their data in a database for research purposes including this study. That research database was approved by the local Ethics Committee from Hospital Universitario Ramón y Cajal (Date of approval: 7-March-2002; Reference number: 12/02). The informed consent was revised and again approved by the same local Institutional Review Board on 2022, March 10.

### Example 1.2. Assays

Serum AMH concentrations were measured using an automated immunochemiluminescent assay on a Cobas e601 ^{®} analyser (Elecsys ^{®}, Roche Diagnostics, Germany). The assay limits of detection and lower limit of quantification were 0.07 and 0.21 pM, respectively. The intra-assay and inter-assay CVs were < 4%. The limit above the measuring range was 164 pM. The relative quantification of plasma miR-142-3p and miR-598-3p was assayed. RNA was extracted from 200 µl plasma using the miRNeasy Serum/plasma Advanced kit (Qiagen #217204, Hilden, Germany). One µg MS2 bacteriophage carrier RNA (Roche #10165948001, Mannheim, Germany) was added during the lysis step. RNA was reverse transcribed into cDNA using the miRCURY LNA RT kit (Qiagen #339340, Maryland, USA). The miRCURY LNA RNA spike-in kit (Qiagen #339390, Maryland, USA) for quality control of the RNA isolation and cDNA synthesis steps was used according to the manufacturer's instructions. The expression of mature miRNAs in plasma was determined with a real-time and miRNA-specific PCR using the miRNA LNA SYBR green PCR kit (Qiagen #339347, Maryland, USA) in a LightCycler ^{®} 480 II, software version 1.5 (Roche Diagnostics GmbH, Mannheim, Germany). To select the most stable endogenous reference miRNAs the inventors used previous miRNA expression data from the laboratory, selecting miR-222-3p and miR-484 as endogenous controls. The geometric mean of the expression of these two reference miRNAs was used as a normalizing factor, with the aim of minimizing analytic variability and obtaining reliable and reproducible results. Relative expression data are shown as arbitrary units using the following transformation [expression = log2^{-ΔCq}], All PCR assays were performed in triplicate for each sample, including miR-142-3p, miR-598-3p, spike-in controls, haemolysis and reference miRNAS.

### Example 1.3. Proton nuclear magnetic resonance spectroscopy metabolomics profiling

Serum samples were shipped to Biosfer Teslab (Metabolomic platform Biosfer Teslab, CIBERDEM, 43206 Tarragona, Spain) in dry ice for the quantification of low-molecular weight (LMW) metabolites by ¹H-NMRS. Serum samples (200 µL) were diluted with 50 µL deuterated water and 300 µL of 50 mM, pH 7.4 phosphate buffer solution (PBS) before analysis. ¹H-NMR spectra were recorded at 300K on a Bruker Avance III 600 spectrometer (Bruker Biospin, Rheinstetten, Germany), operating at a proton frequency of 600 MHz. One-dimensional ¹H pulse experiments were carried out using 1D Carr-Purcell-Meiboom-Gill (CPMG) spectra. Sixty-four transients were collected into 64k data points for each spectrum. The acquired spectra were phased, baseline-corrected and referenced before performing the automatic metabolite profiling of the spectra dataset through and improved adaptation of Dolphin software. The software utilized in the current analysis offers enhanced deconvolution capabilities and high degree of automation.

### Example 1.4. Statistics

The results were expressed as means ± SD and 95% confidence intervals (CI) (lower limit; upper limit), or counts (percentages), as appropriate. The inventors tested the normality of the distribution of continuous variables using the Kolmogorov-Smirnov test. Logarithmic transformation was applied to ensure normality, as needed. Continuous variables were compared by Student's t tests, Mann-Whitney's U tests or univariate general linear models as a function of the homogeneity of variances, distribution, and adjustment by covariates. Since control women were slightly older compared with those women with PCOS, age was included as a covariate in the analyses. Receiver operating characteristics (ROC) curve analysis was used to assess the diagnostic performance of miRNAs and selected metabolites derived from ¹H-NMRS, and to provide the cut-off values showing optimal combinations of sensitivity and specificity.

The inventors also analyzed whether the addition of clinical and biochemical variables to miRNAs and metabolites could improve the accuracy of ROC curves. With this goal, they excluded those variables used for the definition of PCOS (androgens, SHBG, and hirsutism) to avoid spurious improvements in diagnostic performance. First, a binary logistic regression analysis was implemented, introducing having PCOS or being a control as dependent variable. Those miRNAs, ¹H-NMRS-metabolites, and clinical/biochemical variables showing statistically significant differences between patients and controls served as independent variables. Linearity of continuous variables with respect to the logit of dependent variables was assessed via the Box-Tidwell procedure (Box and Tidwell, 1962). Multicollinearity was evaluated by constructing a correlation matrix among predictors. The inventors defined the lack of a strong association by a coefficient below 0.8. Then, they combined those variables retained by the regression model as significant predictors of PCOS and conducted a new ROC analysis in order to obtain the highest AUC values for the discrimination among patients with PCOS and controls. Optimal thresholds were determined by finding the point of the ROC curve closest to the point of best discrimination. The inventors used SPSS Statistics 22.0 (SPSS Ibérica, Madrid, Spain) for the analyses. P < 0.05 was considered statistically significant.

### Example 2. Results

The anthropometrics, sex steroids, and insulin sensitivity indices of non-hyperandrogenic control women and individuals with PCOS are summarized in **Table 1.**

Circulating miR-142-3p was underexpressed in plasma samples of women with PCOS compared to non-hyperandrogenic control women (**Figure 1****,** upper panel). On the contrary, miR-598-3p expression was similar between women with PCOS and non-hyperandrogenic controls (**Figure 1****,** lower panel). The results of ¹H-NMRS metabolomics profiling in women with PCOS and non-hyperandrogenic control women are shown in **Figure 2****.** After adjusting by age, women with PCOS presented with higher fasting circulating concentrations of alanine, leucine, isoleucine, glutamic acid, threonine, acetate and lactate, and lower concentrations of glutamine and acetone, compared to non-hyperandrogenic control women.

Then, the inventors assessed the overall accuracy for the diagnosis of PCOS of miR-142-3p, LMW metabolites with significant differences between PCOS and non-hyperandrogenic control women, LH-to-FSH ratio, and AMH using ROC curve analyses. Among LMW metabolites, only alanine, leucine, isoleucine, threonine, acetate, glutamate, and lactate resulted into statistically significant areas under the ROC curve (AUC_{ROC}) above 0.5 (**Figure 3**).

The inventors performed a binomial logistic regression introducing women status (non-hyperandrogenic control vs. PCOS) as dependent variable, and miR-142-3p, LMW metabolites with statistically significant AUC_{ROC} above 0.5, LH-to-FSH ratio, and AMH as independent variables. Based on the Box-Tidell procedure, all continuous independent variables were found to be linearly related to the logit of the dependent variable. No significant outliers were identified in the model. The logistic regression model was statistically significant χ² (10) = 94.2, *P* < 0.001. The model (Nagelkerke's R² = 0.47) correctly classified 81% of cases. Sensitivity was 85.9%, specificity 60.0%, positive predictive value was 91.0%, and negative predictive value was 47.4%. Of the 10 predictive variables tested, only four were statistically significant: miR-142-3p, isoleucine, acetate, and AMH. A forward stepwise elimination for automated model building using the likelihood-ratio test retained these same four variables as the best predictors of the model. The inventors also excluded five patients with PCOM criteria only based on AMH values in order to avoid spurious associations. In this subset of women, the logistic regression model was also statistically significant χ² (4) = 88.6, *P* < 0.001. The model (Nagelkerke's R² = 0.45) correctly classified 83% of cases. Sensitivity was 86.3%, specificity 65.1%, positive predictive value was 82.4%, and negative predictive value was 49.1%.

The overall discriminatory ability of the binomial logistic regression model was addressed by a new ROC analysis introducing as a test variable the predicted value variable for the model of the four independent variables (**Figure 4**). The AUC ranged from 0.826 to 0.917, which is considered as excellent/outstanding discrimination. The optimal thresholds for these biomarkers were determined by finding the point of the ROC curve closest to the point of best discrimination (**Figure 4****,** red point). Cut-off values of 0.61 log 2^{-ΔCq} for miR-142-3p, 24 µM for isoleucine, 65 µM for acetate, and 23.0 pM for AMH had 65% sensitivity and 95% specificity for diagnosing PCOS. To facilitate the use of the model in a clinical setting, the inventors sought another combination of threshold values that maximized sensitivity. For a 90% sensitivity (**Figure 4****,** dotted green line), the highest specificity value (55%) was obtained with cut-offs of 0.84 log2^{-ΔCq} for miR-142-3p, 22 µM for isoleucine, 25 µM for acetate, and 31.6 pM for AMH.

The inventors also analyzed the performance of the model analysing separately non-obese and obese women. The model correctly classified 82% and 90% of cases, respectively (**Table 2**).

**Table 2. Performance of the discrimination model comprised of four biomarkers in several subset of women and PCOS phenotypes.**

| **Subset of women** | **χ²(4)** | ***P*** | **R²** | **AUC_{ROC} (95% CI)** | **Sensitivity** | **Specificity** | **PPV** | **NPV** |
|---|---|---|---|---|---|---|---|---|
| *Non-obese (BMI < 30 kg*/*m²)* | 68.0 | <0.001 | 0.428 | 0.861 (0.804; 0.918) | 92.6 | 45.7 | 85.7 | 63.6 |
| *Obese (BMI > 30 kg*/*m²)* | 45.0 | <0.001 | 0.611 | 0.936 (0.883; 0.990) | 96.1 | 66.7 | 96.0 | 66.7 |
| *Hyperandrogenic phenotypes* | 101.8 | <0.001 | 0.496 | 0.883 (0.838; 0.927) | 91.8 | 54.7 | 85.3 | 54.7 |
| *Non-ovulatory phenotypes* | 106.7 | <0.001 | 0.473 | 0.877 (0.833; 0.922) | 93.3 | 50.0 | 86.8 | 68.1 |
| *Non-hyperandrogenic phenotypes* | 42.5 | <0.001 | 0.417 | 0.827 (0.753; 0.901) | 64.0 | 79.7 | 71.1 | 73.9 |

| | | | | | | | | |
|---|---|---|---|---|---|---|---|---|
| *Abbreviations* AUC_{ROC}, area under the receiver-operating characteristic curve; BMI, body mass index; NPV, negative predictive value; PPV, positive predictive value; R², Nagelkerke's coefficient. | | | | | | | | |

The subset of women with hyperandrogenic phenotypes includes those individuals with classic and ovulatory PCOS. Non-ovulatory phenotypes are classic PCOS and non-hyperandrogenic PCOS.

When the analysis was conducted separately in women with hyperandrogenic phenotypes, non-ovulatory phenotypes, and non-hyperandrogenic phenotypes - after excluding five women in who PCOM diagnosis was only based on their AMH concentrations, as stated above -the model correctly classified 82%, 84%, and 73% of cases.

## Claims

1. *In vitro* method for the diagnosis and/or prognosis of polycystic ovary syndrome, which comprises assessing the expression level of the microRNA-142-3p in combination with the assessment of the amount of at least a metabolite selected from: isoleucine and/or acetate, in a biological sample obtained from the subject.

2. *In vitro* use of the microRNA-142-3p in combination with isoleucine and/or acetate, or of a kit comprising reagents for assessing the expression level of the microRNA-142-3p in combination with the assessment of the amount of a metabolite selected from: isoleucine and/or acetate, for the diagnosis and/or prognosis of polycystic ovary syndrome.

3. *In vitro* method, or *in vitro* use, according to any of the previous claims, which comprises assessing the expression level of the microRNA-142-3p in combination with the assessment of the amount of the metabolites isoleucine and acetate.

4. *In vitro* method, or *in vitro* use, according to any of the previous claims, which further comprises assessing the concentration of anti-Müllerian hormone.

5. *In vitro* method, or *in vitro* use, according to any of the previous claims, which comprises assessing the expression level of the microRNA-142-3p in combination with the assessment of the amount of the metabolites isoleucine and acetate in combination with the assessment of the concentration of anti-Müllerian hormone.

6. *In vitro* method, or *in vitro* use, according to any of the previous claims, wherein the identification of a lower level of expression of microRNA-142-3p in combination with a higher amount of isoleucine and/or acetate, as compared with a pre-established threshold value measured in control subjects who are not suffering from polycystic ovary syndrome, is an indication that the subject is suffering from polycystic ovary syndrome or has a poor prognosis.

7. *In vitro* method, or *in vitro* use, according to any of the previous claims, wherein the biological sample is selected from: blood, serum or plasma.

8. Kit, suitable for the diagnosis and/or prognosis of polycystic ovary syndrome, which comprises reagents for assessing the expression level of the microRNA-142-3p in combination with reagents for the assessment of the amount of at least a metabolite selected from: isoleucine and/or acetate.

9. Kit, according to claim 8, suitable for the diagnosis and/or prognosis of polycystic ovary syndrome, which further comprises reagents for assessing the concentration of anti-Müllerian hormone.

10. Kit, according to claims 8 or 9, suitable for the diagnosis and/or prognosis of polycystic ovary syndrome, which comprises reagents for assessing the expression level of the microRNA-142-3p in combination with reagents for the assessment of the amount of the metabolites isoleucine and acetate, and reagents for the assessment of the concentration of anti-Müllerian hormone.

11. Hormonal treatment, insulin-sensitizing agents, anti-androgens, ovulation induction agents and/or statins for use in a method for treating polycystic ovary syndrome, wherein the method comprises a step before the treatment which comprises diagnosing polycystic ovary syndrome by following the method of claims 1 to 7.

12. Hormonal treatment, insulin-sensitizing agents, anti-androgens, ovulation induction agents and/or statins for use in a method for treating polycystic ovary syndrome, according to claim 11, wherein the hormonal treatment comprises estrogen and progestin or progestin-only medications; insulin-sensitizing agents that comprise metformin; anti-androgens that comprise spironolactone, finasteride or flutamide; or ovulation induction agents that comprise clomiphene citrate, letrozole or gonadotropins.
